# EUROPEAN PATENT APPLICATION

(11) **EP 2 201 912 A1**
(43) Date of publication of application: **30.06.2010**
(21) Application number: 09178346.4
(22) Date of filing: 08.12.2009
(51) Int. Cl.: A61F 2/40

(54) **Shoulder prosthesis having augmented metaglene component**

(30) Priority: 23.12.2008 US 343272
(71) Applicant: DePuy Products, Inc., Warsaw, IN 46581 (US)
(72) Inventor: Iannotti, Joseph P, Strongsville, OH 44136 (US); Lappin, Kyle E, Fort Wayne, IN 46825 (US); Anthony, Sarah M, Leesburg, IN 46538 (US); Williams, Gerald R Jr, Villanova, PA 19085 (US)
(74) Representative: Belcher, Simon James

(57) **Abstract**

A shoulder prosthesis includes a metaglene component that has (i) a base with a bearing-facing side, a scapula-facing side, and an external coupling surface interposed between them, (ii) an augment attached to the scapula-facing side of the base, and (iii) a post attached to at least one of the base and the augment. The metaglene component has defined therein a passage extending through at least both the base and the post. The metaglene component further having a plurality of fastener holes defined in the base. The shoulder prosthesis includes a bearing component defining a cavity and including a bearing surface, the cavity defining an internal coupling surface, and the bearing-facing side of the mctaglcnc component being located within the cavity so that the external coupling surface of the base is positioned in contact with the internal coupling surface of the bearing component to form a friction fit connection between the bearing component and the metaglene component. The shoulder prosthesis also includes a plurality of fasteners configured to respectively extend through the plurality of fastener holes defined in the base.

## Description

The present invention relates generally to shoulder prostheses, and more particularly to shoulder prostheses configured for use in rotator cuff deficient shoulders.

The rotator cuff is made up of a group of tendons and muscles which includes the deltoid, the supraspinatus, the infraspinatus, the infrascapular, and the smaller round. When massive rupture occurs of the rotator cuff, only the deltoid muscle remains functional which is insufficient to enable proper operation of the shoulder joint. Moreover, improper operation of the shoulder joint due to massive rotator cuff rupture when left untreated will cause erosion or other defects in the subchondral surface of the glenoid. Thus, it is common that a patient who is being treated for a rotator cuff deficiency will also have erosion or other defects of the subchondral surface of the glenoid.

Certain procedures have been used to treat rotator cuff deficient shoulders which have the above described glenoid erosion or defects. For example, the bone of the glenoid may be prepared asymmetrically to create an appropriately configured support to receive a typical metaglene component of a shoulder prosthesis. Asymmetric preparation of bone involves removing more bone from one side of the glenoid in comparison to another in order to create an even support surface for receipt of the metaglene component. In another example, a bone graft is utilized in conjunction with implantation of a standard metaglene component, the bone graft being configured to fill the eroded or defected area of the glenoid so that the implanted metaglene component is appropriately supported. Yet another example involves interposition shoulder arthroplasty in which new tissue is placed between the damaged surfaces of the joint. In interposition shoulder arthroplasty, a tissue-type graft is sutured over the eroded or defected area of the glenoid so as to ease the pain of the damaged joint while allowing the shoulder joint to retain some function. Interposition shoulder arthroplasty is typically a temporary solution to shoulder joint deficiency, and standard shoulder reconstruction will typically follow after several months.

Each of these treatments has significant drawbacks. For example, implanting a metaglene component in bone that has been prepared asymmetrically results in healthy bone stock being sacrificed. Use of a bone graft in conjunction with a metaglene component may have complications due to graft non-union and not all patients have adequate bone stock available for such a procedure. Interposition shoulder arthroplasty tends to be a short term solution that masks the shoulder joint deficiency, only to be followed some time later by more invasive conventional shoulder reconstruction in which humeral and glenoid components are implanted. This two step process results in more risk and inconvenience to the patient since two surgical procedures are involved.

In one aspect, the invention provides a shoulder prosthesis having a metaglene component which includes (i) a base having a bearing-facing side, a scapula-facing side, and an external coupling surface interposed between them, (ii) an augment attached to the scapula-facing side of the base, and (iii) a post attached to at least one of the base and the augment. The metaglene component has defined therein a passage extending through at least both the base and the post. The metaglene component further has a plurality of fastener holes defined in the base. The shoulder prosthesis further includes a bearing component defining a cavity and including a bearing surface, the cavity defining an internal coupling surface, and the bearing-facing side of the metaglene component being located within the cavity so that the external coupling surface of the base is positioned in contact with the internal coupling surface of the bearing component to form a friction fit connection between the bearing component and the metaglene component. The shoulder prosthesis includes a plurality of fasteners configured to respectively extend through the plurality of fastener holes defined in the base.

In another aspect, the invention provides a shoulder prosthesis which includes a metaglene component having (i) a base having a bearing-facing side and a scapula-facing side, the base further having defined therein a plurality of fastener holes defined therein, (ii) an augment extending from the scapula-facing side of the base, and (iii) a post extending from at least one of the base and the augment. The shoulder prosthesis further includes a bearing component defining a cavity and including a bearing surface, and the bearing-facing side of the metaglene component being located within the cavity. In addition, the shoulder prosthesis includes a plurality of fasteners configured to respectively extend through the plurality of fastener holes defined in the base.

Preferably, the base of the metaglene component includes an external peripheral wall surface interposed between the bearing-facing side and the scapula-facing side, the cavity of the bearing component defining an internal wall surface, and the external peripheral wall surface is positioned in contact with the internal wall surface to form a friction fit connection between the bearing component and the metaglene component.

Preferably, the metaglene component has defined therein a passage extending through at least both the base and the post, the passage defined in the metaglene component defines an internally threaded wall portion, the bearing component further includes a screw that is rotatable in relation to the bearing surface, and the screw defines an externally threaded wall portion that meshingly engages the internally threaded wall portion when said bearing component is coupled to said metaglene component.

Preferably, the central passage defined in the metaglene component further extends through the augment.

Preferably, the augment defines an external sidewall that is aligned with the external peripheral wall surface of the base.

Preferably, the external peripheral wall surface of the base extends completely circumferentially around the metaglene component, and the external sidewall of the augment extends circumferentially for more than a half of the circumferential extent of the external peripheral wall surface of the base.

Preferably, at least one of the plurality of fastener holes includes an end opening that is at least partially defined in the external sidewall of the augment.

The post can extend only from the augment. The post can extend from both the base and the augment.

Preferably, all of the plurality of fastener holes are further defined in the augment.

Preferably, the shoulder prosthesis includes a humeral component having (i) a stem configured to be implanted within an intramedullary canal of a humerus, and (ii) another bearing surface configured to mate with said bearing surface of said bearing component.

The shoulder prosthesis of the invention can be used in a rotator cuff deficient shoulder which exhibits glenoid erosion or defects. The prosthesis of the invention can be used in a rotator cuff deficient shoulder which exhibits glenoid erosion or defects in such a way that conserves healthy bone stock. The shoulder prosthesis of the invention can be used in a rotator cuff deficient shoulder which exhibits glenoid erosion or defects, without necessarily having to implant bone graft in conjunction with the shoulder prosthesis. The shoulder prosthesis of the invention can be used in a rotator cuff deficient shoulder which exhbits glenoid erosion or defects, without necessarily having to employ a two stage surgical approach to restoring proper function of the shoulder joint.

Embodiments of the invention are described below by way of example with reference to the accompanying drawings, in which:
Fig. 1 is a perspective view of a shoulder prosthesis according to the invention, implanted in a scapula and a humerus of a patient to form a joint between them;
Fig. 2 is a cross sectional view of a glenoid component of the shoulder prosthesis of Fig. 1 taken with respect to the direction shown by arrows 2-2 of Fig. 5;
Fig. 3 is a cross sectional view of the glenoid component of the shoulder prosthesis of Fig. 1 taken with respect to the direction shown by arrows 3-3 of Fig. 5;
Fig. 4 is an exploded cross sectional view of the glenoid component of Figs. 2 and 3, shown with the bone fasteners removed for clarity;
Fig. 5 is a top elevational view of the metaglene component of the glenoid component of Figs. 2 to 4;
Fig. 6 is a side elevational view of the metaglene component of the glenoid component of Figs. 2 to 4;
Fig. 7 is a bottom perspective view of the metaglene component of the glenoid component of Figs. 2 to 4;
Fig. 8 is a side elevational view of an alternative embodiment of a metaglene component that is configured to be used with the shoulder prosthesis of Fig. 1;
Fig. 9 is a bottom perspective view of the metaglene component of Fig. 8;
Fig. 10 is a top perspective view of the metaglene component of the glenoid component of Fig. 8;
Fig. 11 is a side elevational view of yet another alternative embodiment of a metaglene component that is configured to be used with the shoulder prosthesis of Fig. 1;
Fig. 12 is a bottom perspective view of the metaglene component of Fig. 11;
Fig. 13 is a side elevational view of still another alternative embodiment of a metaglene component that is configured to be used with the shoulder prosthesis of Fig. 1;
Fig. 14 is a bottom perspective view of the metaglene component of Fig. 13;
Fig. 15 is a side elevational view of another alternative embodiment of a metaglene component that is configured to be used with the shoulder prosthesis of Fig. 1; and
Fig. 16 is a bottom perspective view of the metaglene component of Fig. 15.

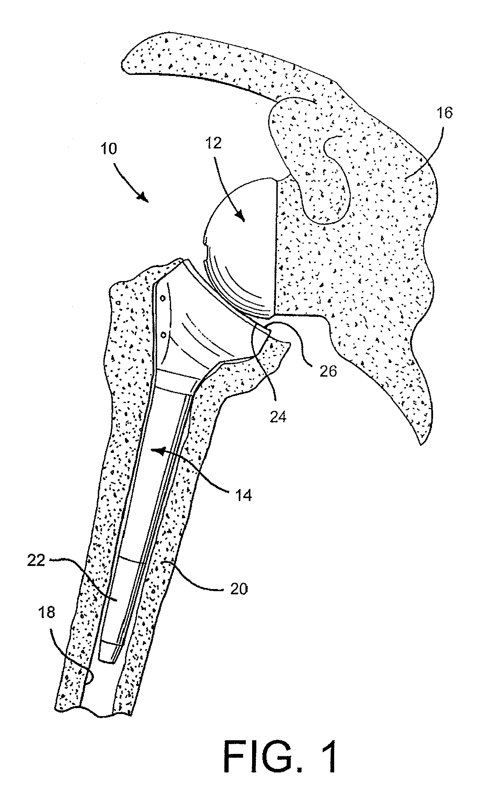

Referring to the drawings, Fig. 1 shows a shoulder prosthesis 10 which includes a glenoid component 12 and a humeral component 14 that are configured to cooperate with each other to form a shoulder joint. The glenoid component 12 can be attached to a glenoid of a scapula 16, while the humeral component can be implanted in an intramedullary canal 18 of a humerus 20 as shown in Fig. 1.

The humeral component 14 includes a stem 22 that is configured to be received in the intramedullary canal 18 as shown in Fig. 1. The humeral component 14 further includes a bearing component 24 that has a bearing surface 26.

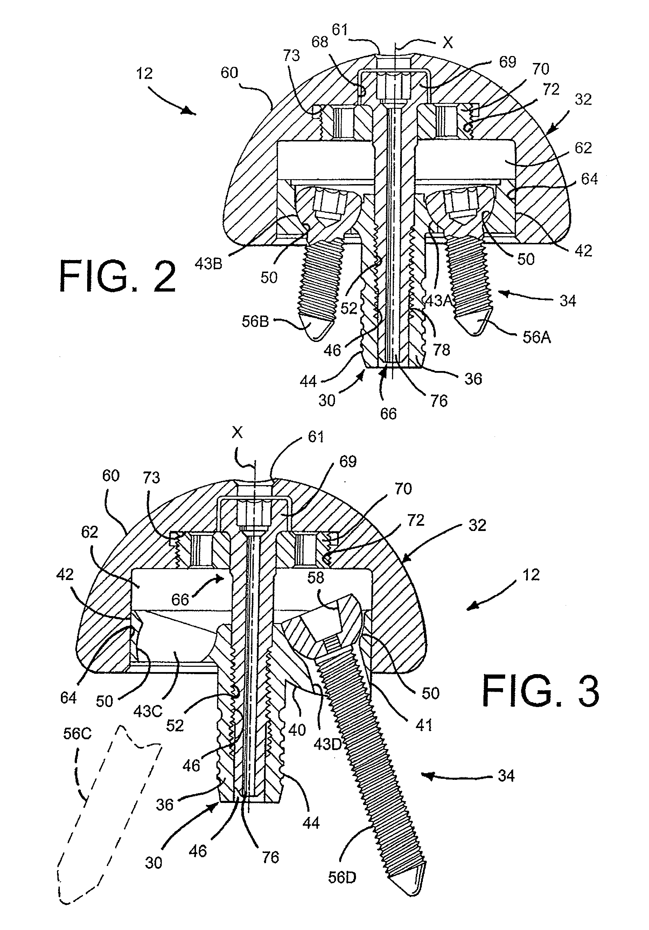

The glenoid component 12 of the shoulder prostheses 10 is shown in more detail in Figs. 2 to 7. In particular, the glenoid component 12 includes a metaglene component 30, a bearing component 32, and a plurality of fasteners 34. The metaglene component 30 is configured to be attached to the glenoid of the scapula 16 with the fasteners 34, while the bearing component 32 is configured to be coupled to the metaglene component 30 as shown in Figs. 2 and 3.

The metaglene component 30 includes a base 36 having a bearing-facing side 38 and a scapula-facing side 39. The base 36 further has an external peripheral wall surface 42 that defines an external coupling surface that is interposed between the bearing-facing side 38 and the scapula-facing side 39 as shown in Figs. 2 to 4. The external peripheral wall surface 42 extends completely (that is through an angle of 360°) around the periphery of the metaglene component. The base 36 has defined therein a plurality of fastener holes 43A, 43B, 43C, and 43D. The fastener holes 43A, 43B, 43C, and 43D are defined, in part, by walls having concave bearing seats 50.

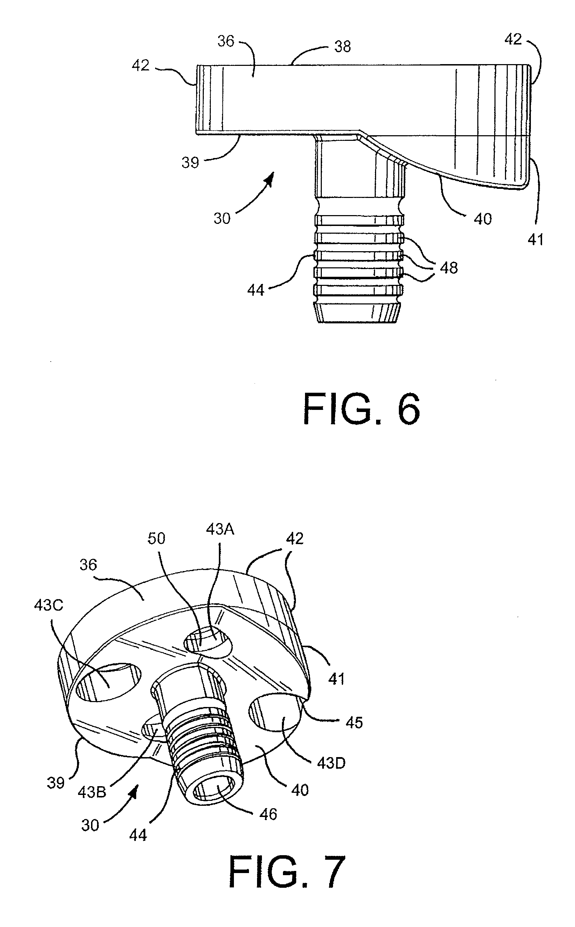

The metaglene component 30 further includes an augment 40 attached to the scapula-facing side 39 of the base 36. The augment 40 is attached to the base 36 by being integrally formed with it. Alternatively, the augment 40 may be removably attached to the base 36 by a coupling mechanism (not shown). For example, the coupling mechanism may include a post (not shown) attached to the augment and a recess (not shown) formed in the base, in which the post and recess mate in a friction fit manner to secure the augment to the base. The augment 40 defines an external sidewall 41 that is aligned with the external peripheral wall surface 42 of the base 36 (see, e.g., Figs. 6 and 7). The fastener hole 43D includes a distal end opening 45 that is partially defined in the external side wall 41 as shown in Fig. 7 (see also Fig. 3).

In the embodiment of the metaglene component 30 shown in Figs. 1 to 7, the external sidewall 41 of the augment 40 extends circumferentially for a half of the extent of the external coupling surface 42 of the base 36. In other words, the external sidewall 41 of the augment 40 extends circumferentially through an angle of 180° around the periphery of the base 36. In other embodiments discussed below, the external sidewall of the augment extends circumferentially for more than a half of the extent of the external coupling surface of the base. Alternatively, as discussed below, the external sidewall of the augment may extend circumferentially for less than half of the extent of the external coupling surface of the base. Each of these constructions has particular benefits.

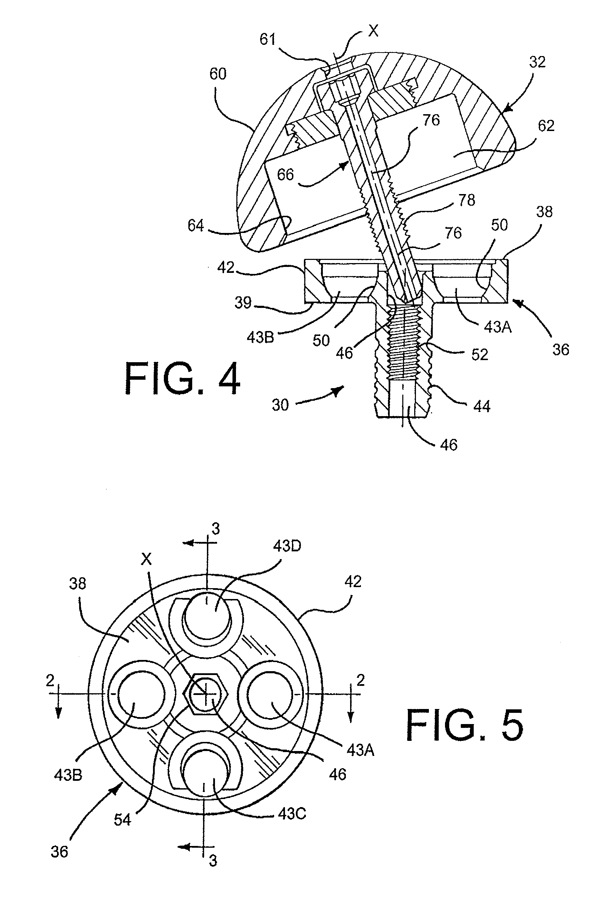

The metaglene component 30 also includes a post 44 extending from the base 36 and the augment 40. The post 44 is attached to the base 36 and the augment 40 by being integrally formed therewith. A plurality of ribs 48 are defined on the post 44. The post 44 has defined therein a central passage 46. The post 44 extends through the base 36 as shown in Figs. 2 to 5. The post 44 includes an internally threaded wall portion 52 as shown in Figs. 2 to 4. The base 36 has a hexagonal-shaped opening 54 defined therein that is aligned with the central passage 46 as shown in Fig. 5.

As discussed above, the glenoid component 12 further includes fasteners 56A, 56B, 56C, and 56D. The fasteners 56A, 56B are configured to respectively extend through the fastener holes 43A, 43B, while the fasteners 56C, 56D are configured to respectively extend through the fastener holes 56A, 56B. Each of the fasteners 56A, 56B, 56C, and 56D includes a head having a convex surface configured to be matingly received by a respective concave bearing seat 50 of a respective fastener hole as shown in Figs. 2 and 3. Each of the fasteners 56A, 56B, 56C, and 56D is configured to be adjustable to any one of a variety of angles with respect to the base 36 due to the spherical shape of both the fastener heads and the concave bearing seats 50. The fasteners 56C, 56D are locking fasteners since advancement of an expander (not shown) into a head recess 58 (see Fig. 3) of its respective head causes the fastener head to expand thereby locking the head and thus the fastener 56C, 56D to the base 36. The fasteners 56A, 56B are non-locking fasteners. Any or all if the fasteners 56A, 56B, 56C, and 56D may be locking fasteners or non-locking fasteners. For example, the fasteners 56A, 56B may be locking fasteners, while the fasteners 56C, 56D may be non-locking fasteners. Any combination of locking and non-locking fasteners may be used in the metaglene component 30.

As shown in Figs. 2 to 4, the bearing component 32 of the glenoid component 12 includes a substantially hemispherical bearing surface 60 that defines an axis X. The bearing surface 60 has an access opening 61 defined therein. The bearing surface 60 is configured to mate with the bearing surface 26 of the bearing component 24 of the humeral component 14 as shown in Fig. 1. In particular, the bearing surface 60 defines a convex surface and the bearing surface 26 defines a concave surface which is configured to receive the convex bearing surface. Alternatively, the bearing surface 60 may be configured to define a concave surface and the bearing surface 26 may be configured to define a convex surface which is configured to receive the alternative concave bearing surface.

In addition, the bearing component 32 defines a cavity 62. The cavity 62 defines an internal wall surface 64 that defines an internal coupling surface. The internal wall surface 64 extends completely (that is through an angle of 360°) around the cavity 62. The external peripheral wall surface 42 is positioned in contact with the internal wall surface 64 to form a friction fit connection between the bearing component 32 and the metaglene component 30 as shown in Figs. 2 and 3. In order to facilitate the friction fit connection, both the external peripheral wall surface 42 and the internal wall surface 64 are tapered so that the surfaces 42, 64 when joined together form a Morse taper connection.

The bearing component 32 further includes a screw 66 that is aligned with the axis X. The bearing component 32 further defines a space 68 in which a head 69 of the screw 66 is retained by a washer 70. In particular, the bearing component 32 further defines an internally threaded wall 72 which meshes with external threads 73 of the washer 70. So retained, the screw 66 is free to rotate in relation to the bearing surface 60. The screw 66 includes a longitudinal axial channel 76 as shown in Figs. 2 to 4. The screw 66 also includes an externally threaded portion 78 that is configured to meshingly engage the internally threaded portion 52 of the post as shown in Figs. 2 and 3. Relevant features of the structure and the operation of the screw and related components are discussed in US-6953478.

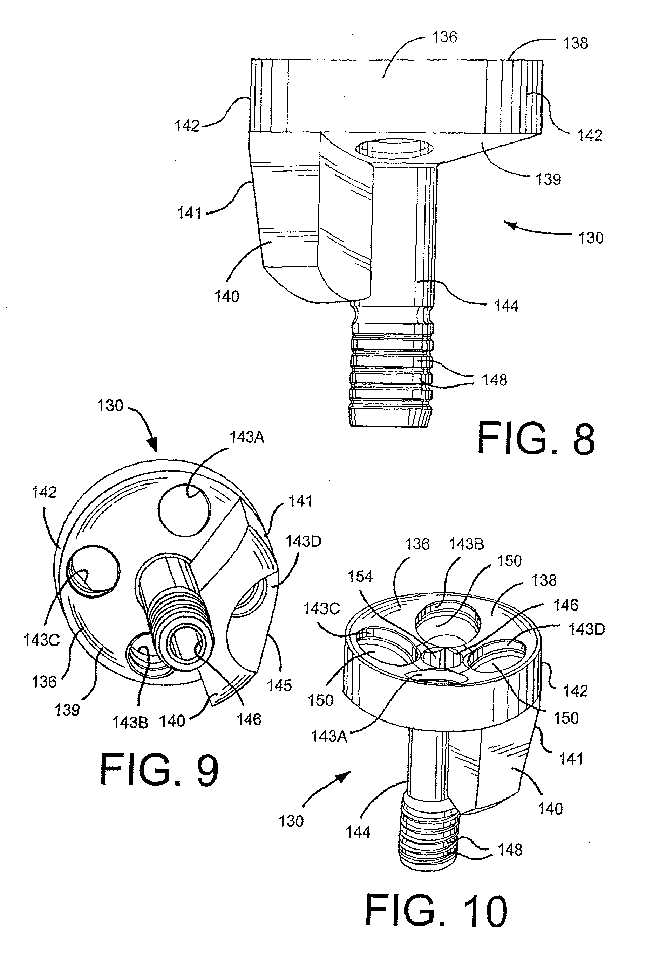

Figs. 8 to 10 show an embodiment of a metaglene component 130 that is configured and used in a manner exactly the same as the metaglene component 30 described above, with one exception. The exception relates to the configuration of the augment of the metaglene component. In particular, the metaglene component 130 includes an augment 140 as shown in Figs. 8 to 10. The augment 140 has a different configuration in relation to the augment 40 of the metaglene component 30 shown in Figs. 7 and 8.

The metaglene component 130 includes a base 136 having a bearing-facing side 138 and a scapula-facing side 139. The base 136 further has an external peripheral wall surface 142 that defines an external coupling surface that is interposed between the bearing-facing side 138 and the scapula-facing side 139 as shown in Figs. 8 to 10. The external peripheral wall surface 142 extends completely (that is through an angle of 360°) around the periphery of the metaglene component. The base 136 has defined therein a plurality of fastener holes 143A, 143B, 143C, and 143D. The fastener holes 143A, 143B, 143C, and 143D are defined by walls having concave bearing seats 150.

The augment 140 of the metaglene component 130 is attached to the scapula-facing side 139 of the base 136. The augment 140 is attached to the base 136 by being integrally formed therewith. Alternatively, the augment 140 may be removably attached to the base 136 by a coupling mechanism (not shown). The augment 140 defines an external sidewall 141 that is aligned with the external peripheral wall surface 142 of the base 136. The fastener hole 143D includes a distal end opening 145 that is partially defined in the external side wall 141 as shown in Fig. 9.

In the embodiment of the metaglene component 130 shown in Figs. 8 to 10, the external sidewall 141 of the augment 140 extends circumferentially for a third of the extent of the external coupling surface 142 of the base 136 (best shown in Fig. 9). In other words, the external sidewall 141 of the augment 140 extends circumferentially for 120° around the periphery of the base 136.

The metaglene component 130 also includes a post 144 extending from the base 136. The post 144 is attached to the base 136 by being integrally formed therewith. A plurality of ribs 148 are defined on the post 144. The post 144 has defined therein a central passage 146. The central passage 146 extends through the base 136. The post 144 includes an internally threaded wall portion (not shown). The base 136 has a hexagonal-shaped opening 154 defined therein that is aligned with the central passage 146.

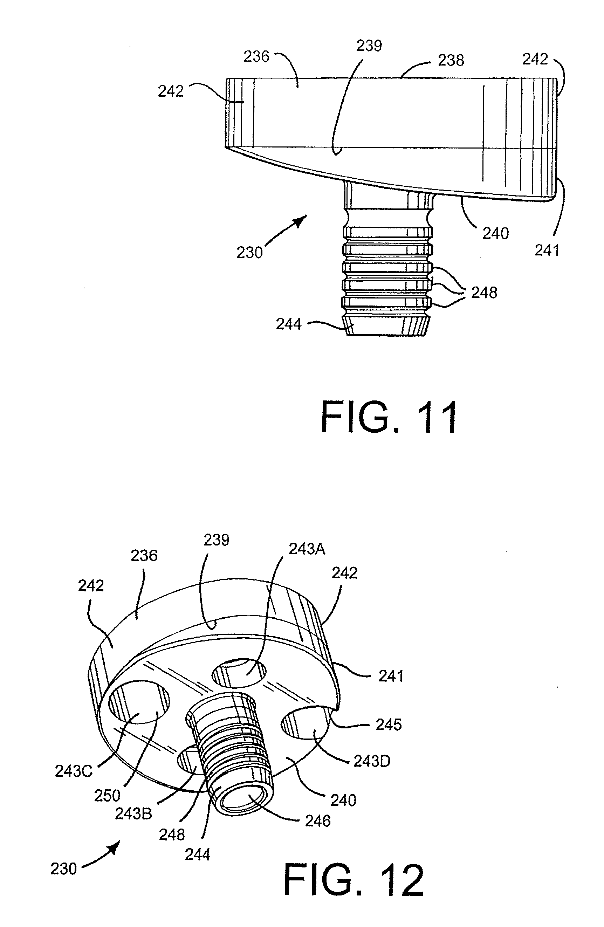

Figs. 11 and 12 show a metaglene component 230 which can be used in a manner exactly the same as the metaglene component 30 described above, with an exception similar to the one described above. In particular, the metaglene component 230 includes an augment 240 (shown in Figs. 11 and 12) that is differently configured in relation to the augment 40 of the metaglene component 30 shown in Figs. 7 and 8.

The metaglene component 230 includes a base 236 having a bearing-facing side 238 and a scapula-facing side 239. The base 236 further has an external peripheral wall surface 242 that defines an external coupling surface that is interposed between the bearing-facing side 238 and the scapula-facing side 239 as shown in Figs. 11 and 12. The external peripheral wall surface 242 extends completely (that is through an angle of 360°) around the periphery of the metaglene component. The base 236 has defined therein a plurality of fastener holes 243A, 243B, 243C, and 243D. Due to the configuration of the augment in this embodiment, each of the plurality of fastener holes 243A, 243B, 243C, and 243D is further defined in the augment 240 as shown in Fig. 12. The fastener holes 243A, 243B, 243C, and 243D are defined, in part, by walls having concave bearing seats 250.

The augment 240 of the metaglene component 230 is attached to the scapula-facing side 239 of the base 236. The augment 240 is attached to the base 236 by being integrally formed therewith. Alternatively, the augment 240 may be removably attached to the base 236 by a coupling mechanism (not shown). The augment 240 defines an external sidewall 241 that is aligned with the external peripheral wall surface 242 of the base 236. The fastener hole 243D includes a distal end opening 245 that is partially defined in the external side wall 241 as shown in Fig. 12.

In the embodiment of the metaglene component 230 shown in Figs. 11 and 12, the external sidewall 241 of the augment 240 extends circumferentially for the entire extent of the external coupling surface 242 of the base 236. In other words, the external sidewall 241 of the augment 240 extends circumferentially through an angle of 360° around the periphery of the base 236.

The metaglene component 230 also includes a post 244 extending from entirely from the augment 240. The post 244 is attached to the augment 240 by being integrally formed therewith. A plurality of ribs 248 are defined on the post 244. The post 244 has defined therein a central passage 246. The central passage 246 extends through the base 236. Note that since the central passage 246 extends entirely from the augment 240, the central passage 246 additionally extends though the augment 240. The post 244 includes an internally threaded wall portion (not shown). The base 236 has a hexagonal-shaped opening (not shown) defined on the bearing-facing side 238 that is aligned with the central passage 246.

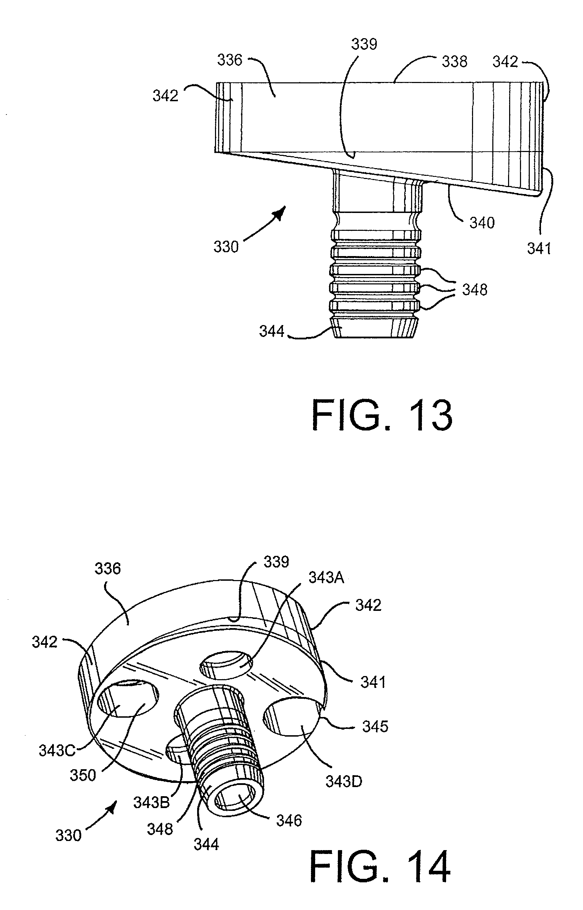

Figs. 13 and 14 show a metaglene component 330 which can be used in a manner exactly the same as the metaglene component 30 described above, with an exception similar to the ones described above. In particular, the metaglene component 330 includes an augment 340 as shown in Figs. 13 and 14 which is configured in a different manner in relation to the augment 40 of the metaglene component 30 shown in Figs. 7 and 8.

The metaglene component 330 includes a base 336 having a bearing-facing side 338 and a scapula-facing side 339. The base 336 further has an external peripheral wall surface 342 that defines an external coupling surface that is interposed between the bearing-facing side 338 and the scapula-facing side 339 as shown in Figs. 13 and 14. The external peripheral wall surface 342 extends completely (that is through an angle of 360°) around the periphery of the metaglene component. The base 336 has defined therein a plurality of fastener holes 343A, 343B, 343C, and 343D. Due to the configuration of the augment in this embodiment, each of the plurality of fastener holes 343A, 343B, 343C, and 343D is further defined in the augment 340 as shown in Fig. 12. The fastener holes 343A, 343B, 343C, and 343D are defined, in part, by walls having concave bearing seats 350.

The augment 340 of the metaglene component 330 is attached to the scapula-facing side 339 of the base 336. The augment 340 is attached to the base 336 by being integrally formed therewith. Alternatively, the augment 340 may be removably attached to the base 336 by a coupling mechanism (not shown). The augment 340 defines an external sidewall 341 that is aligned with the external peripheral wall surface 342 of the base 336. The fastener hole 343D includes a distal end opening 345 that is partially defined in the external side wall 341 as shown in Fig. 14.

In the embodiment of the metaglene component 330 shown in Figs. 13 and 14, the external sidewall 341 of the augment 340 extends circumferentially for the entire extent of the external coupling surface 342 of the base 336. In other words, the external sidewall 341 of the augment 340 extends circumferentially through an angle of 360° around the periphery of the base 336.

The metaglene component 330 also includes a post 344 extending from entirely from the augment 340. The post 344 is attached to the augment 340 by being integrally formed therewith. A plurality of ribs 348 are defined on the post 344. The post 344 has defined therein a central passage 346. The central passage 346 extends through the base 336. Note that since the central passage 346 extends entirely from the augment 340, the central passage 346 additionally extends though the augment 340. The post 344 includes an internally threaded wall portion (not shown). The base 336 has a hexagonal-shaped opening (not shown) defined on the bearing-facing side 338 that is aligned with the central passage 346.

The metaglene component 330 of the embodiment shown in Figs. 13 and 14 is structurally the same as the metaglene component 230 of the embodiment shown in Figs. 11 and 12, except that the scapula-facing side of the augment 340 of the metaglene component 330 is configured to be generally planar (best seen in Fig. 13) while the scapula-facing side of the augment 240 of the metaglene component 230 possesses a convex configuration (best seen in Fig. 11).

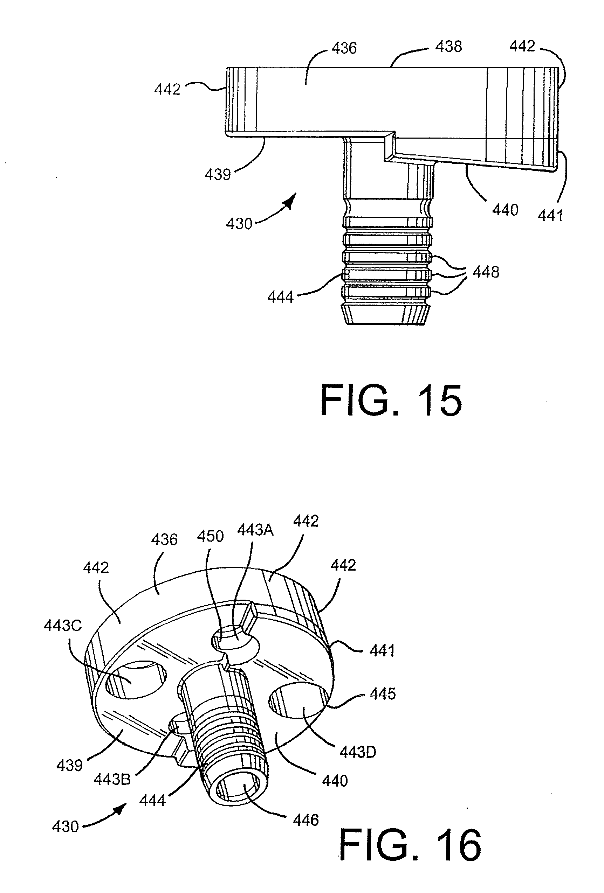

Figs. 15 and 16 show a metaglene component 430 which can be used in a manner exactly the same as the metaglene component 30 described above, with an exception similar to the ones described above. In particular, the metaglene component 430 includes an augment 440 as shown in Figs. 15 and 16 which is configured in a different manner in relation to the augment 40 of the metaglene component 30 shown in Figs. 7 and 8.

The metaglene component 430 includes a base 436 having a bearing-facing side 338 and a scapula-facing side 439. The base 436 further has an external peripheral wall surface 442 that defines an external coupling surface that is interposed between the bearing-facing side 438 and the scapula-facing side 439 as shown in Figs. 15 and 16. The external peripheral wall surface 442 extends completely (that is through an angle of 360°) around the periphery of the metaglene component. The base 436 has defined therein a plurality of fastener holes 443A, 443B, 443C, and 443D. The fastener holes 443A, 443B, 443C, and 443D are defined by walls having concave bearing seats 450. Due to the configuration of the augment in this embodiment, the fastener hole 434C is only defined in the base 436, while each of the plurality of fastener holes 343A, 343B, and 343D is defined in both the base 436 and the augment 340 as shown in Fig. 16. Note that the entire distal end opening 445 of the fastener opening 443D is defined in the augment 440. In contrast, the entire distal end opening of the fastener opening 443C is defined in the base 436. Also, the distal end openings of the fastener openings 443A, 443B are defined by both the base 436 and the augment 440 as shown in Fig. 16.

The augment 440 of the metaglene component 430 is attached to the scapula-facing side 439 of the base 436. The augment 440 is attached to the base 436 by being integrally formed therewith. Alternatively, the augment 440 may be removably attached to the base 436 by a coupling mechanism (not shown). The augment 440 defines an external sidewall 441 that is aligned with the external peripheral wall surface 442 of the base 436. The fastener hole 443D includes a distal end opening 445 that is partially defined in the external side wall 441 as shown in Fig. 16.

In the embodiment of the metaglene component 430 shown in Figs. 15 and 16, the external sidewall 441 of the augment 440 extends circumferentially for half of the extent of the external coupling surface 442 of the base 436. In other words, the external sidewall 441 of the augment 440 extends circumferentially through an angle of 180 around the periphery of the base 436.

The metaglene component 430 also includes a post 444 extending from both the base 436 and the augment 440. The post 444 is attached to the base 436 and the augment 440 by being integrally formed therewith. A plurality of ribs 448 are defined on the post 444. The post 444 has defined therein a central passage 446. The central passage 446 extends through the base 436. Note that since the central passage 446 extends partially from the augment 440, the central passage 446 additionally extends though the augment 440. The post 444 includes an internally threaded wall portion (not shown). The base 436 has a hexagonal-shaped opening (not shown) defined on the bearing-facing side 438 that is aligned with the central passage 446.

## Claims

1. A shoulder prosthesis comprising:
a metaglene component having (i) a base having a bearing-facing side, a scapula-facing side, and an external coupling surface interposed between them, (ii) an augment attached to the scapula-facing side of the base, and (iii) a post attached to at least one of the base and the augment, the metaglene component having defined therein a passage extending through at least both the base and the post, and the metaglene component further having a plurality of fastener holes defined in the base;
a bearing component defining a cavity and including a bearing surface, the cavity defining an internal coupling surface, and the bearing-facing side of the metaglene component being located within the cavity so that the external coupling surface of the base is positioned in contact with the internal coupling surface of the bearing component to form a friction fit connection between the bearing component and the metaglene component; and
a plurality of fasteners configured to respectively extend through the plurality of fastener holes defined in the base.

2. The shoulder prosthesis of claim 1, in which the passage defined in the metaglene component further extends through the augment.

3. The shoulder prosthesis of claim 1, in which the passage defined in the metaglene component defines an internally threaded wall portion, the bearing component includes a screw that is rotatable in relation to the bearing surface, and the screw defines an externally threaded wall portion that meshingly engages the internally threaded wall portion when said bearing component is coupled to said metaglene component.

4. The shoulder prosthesis of claim 1, in which the metaglene component has a polygonal shaped opening defined in the bearing-facing side of the base that is aligned with the passage.

5. The shoulder prosthesis of claim 1, in which the augment defines an external sidewall that is aligned with the external coupling surface of the base.

6. The shoulder prosthesis of claim 5, in which the external coupling surface of the base extends completely around the metaglene component, and the external sidewall of the augment extends circumferentially for more than a half of the extent of the external coupling surface of the base.

7. The shoulder prosthesis of claim 5, in which at least one of the plurality of fastener holes includes an end opening that is at least partially defined in the external sidewall of the augment.

8. The shoulder prosthesis of claim 1, in which the post extends only from the augment, or extends from both the base and the augment.

9. The shoulder prosthesis of claim 1, in which the external coupling surface of the base defines a first tapered surface, the internal coupling surface of the bearing component defines a second tapered surface, and the first tapered surface and the second tapered surface cooperate to form a friction fit connection between them.

10. The shoulder prosthesis of claim 1, in which the bearing surface of the bearing component is one of a concave bearing surface and a convex bearing surface.

11. The shoulder prosthesis of claim 1, in which the base and the augment are integrally formed together.

12. The shoulder prosthesis of claim 1, in which the base, the augment, and the post are integrally formed together.

13. The shoulder prosthesis of claim 1, in which at least one of the plurality of fastener holes is further defined in the augment.

14. The shoulder prosthesis of claim 1, in which all of the plurality of fastener holes are further defined in the augment.

15. The shoulder prosthesis of claim 1, which includes a humeral component having
(i) a stem configured to be implanted within an intramedullary canal of a humerus, and
(ii) another bearing surface configured to mate with said bearing surface of said bearing component.
